# EUROPÄISCHE PATENTSCHRIFT

(11) **EP 1 891 992 B1**
(45) Veröffentlichungstag und Bekanntmachung des Hinweises auf die Patenterteilung: **04.12.2013**
(21) Anmeldenummer: 07013445.7
(22) Anmeldetag: 10.07.2007
(51) Int. Cl.: A61L 31/10, A61L 31/16

(54) **Biodegradierbarer Stent mit einer aktiven Beschichtung**
Biodegradable stent with an active coating
Stent biodégradable doté d'un revêtement actif

(30) Priorität: 07.08.2006 DE 102006038236
(43) Veröffentlichungstag der Anmeldung: 27.02.2008
(73) Patentinhaber: Biotronik VI Patent AG, 6341 Baar (CH)
(72) Erfinder: Tittelbach, Michael, 90429 Nürnberg (DE)
(74) Vertreter: Lindner-Vogt, Karin L.

(56) Entgegenhaltungen:
- WO-A-2005/075005
- US-A1- 2002 155 212
- US-A1- 2005 283 229
- US-A1- 2006 136 051

## Beschreibung

Die Erfindung betrifft einen biodegradierbaren Stent mit einer aktiven Beschichtung.

Seit nunmehr zwei Jahrzehnten hat sich in der Medizintechnik die Implantation von endovaskulären Stützsystemen als eine der wirkungsvollsten therapeutischen Maßnahmen bei der Behandlung von Gefäßerkrankungen etabliert. Beispielsweise bei der interventionellen Therapie der stabilen und instabilen Angina pectoris hat die Einführung von Stents zu einer deutlichen Reduktion der Restenoserate und damit zu besseren Langzeitresultaten geführt. Ursächlich für den Nutzen der Stentimplantation bei vorgenannter Indikation ist der höhere primäre Lumengewinn. Durch den Einsatz von Stents kann zwar ein für den Therapieerfolg primär notwendiger optimaler Gefäßquerschnitt erreicht werden, allerdings indiziert die dauerhafte Anwesenheit eines derartigen Fremdkörpers körpereigene Prozesse, die zu einem allmählichen Zuwachsen des Stentlumens führen können.

Jedes der Dokumente US 2005/283229 A1 und WO 2005/075005 A1 offenbart einen Stent mit einem Grundkörper aus einem biodegradierbaren Material und einer auf dem Grundkörper aufgebrachten aktiven Beschichtung, die eine biodegradierbare Trägermatrix und zumindest eine in die Trägermatrix eingebettete pharmazeutisch aktive Substanz umfasst. Die Abbaugeschwindigkeit der aktiven Beschichtung ist geringer als die Abbaugeschwindigkeit des Grundkörpers. Ferner offenbart WO 2005/075005 A1 eine oder mehrere Kavitäten im Grundkörper, die die Trägermatrix enthalten, und US 2005/283229 A1 offenbart Poren in dem Trägermatrix.

Ein Ansatzpunkt zur Lösung dieser Problematik besteht darin, den Stent aus einem biodegradierbaren Werkstoff zu fertigen. Zur Realisation derartiger biodegradierbarer Implantate stehen dem Medizintechniker verschiedenstartige Werkstoffe zur Verfügung. Neben zahlreichen Polymeren, die häufig zur besseren Biokompatibilität natürlichen Ursprungs oder zumindest an natürliche Verbindungen angelehnt sind, werden in jüngster Zeit metallische Werkstoffe mit ihren für die Implantate wesentlich günstigeren mechanischen Eigenschaften favorisiert. Besondere Beachtung finden in diesem Zusammenhang magnesium-, eisen- und wolframhaltige Werkstoffe.

Ein zweiter Ansatzpunkt zur Minderung der Restenosegefahr liegt in der lokalen Applikation von pharmazeutischen Substanzen (Wirkstoffen), die auf zellulärer Ebene den verschiedenen Mechanismen der pathologischen Gefäßveränderungen entgegenwirken sollen bzw. den Heilungsverlauf unterstützen sollen. Die pharmazeutischen Substanzen werden in der Regel in eine Trägermatrix eingebettet, um (i) eine Elutionscharakteristik der pharmazeutischen Substanz zu beeinflussen, (ii) ein Anheften der Beschichtung an der Implantatoberfläche zu unterstützen und (iii) die Herstellung der Beschichtung, insbesondere das Aufbringen einer definierten Menge an Wirkstoffen zu optimieren.

Als Trägermatrix haben sich Materialien unterschiedlichster Ausführung bewährt. Es kann zwischen permanenten Beschichtungen und Beschichtungen aus einer biodegradierbaren Trägermatrix unterschieden werden. Letztere greifen zumeist auf Polymere biologischen Ursprungs zurück. Trägermatrix, pharmazeutische Substanz und ggf. weitere Hilfsstoffe bilden zusammen eine so genannte "aktive Beschichtung" auf dem Implantat aus.

Es bietet sich an, die beiden vorgenannten Ansatzpunkte zu kombinieren, um die Restenoserate weiter zu senken und den Heilungsprozess zu unterstützen. Insbesondere dürfte dabei eine Kombination aus biodegradierbaren Implantatsgrundkörper mit einer ebenfalls biodegradierbaren aktiven Beschichtung vorteilhaft sein.

In ersten Versuchen der Anmelderin hat es sich nun gezeigt, dass die aktive Beschichtung einen erheblichen Einfluss auf das Degradationsverhalten des Implantatsgrundkörpers hat; Bereiche die großflächig von der aktiven Beschichtung bedeckt werden, sind dem Körpermedium, in der Regel Blut, nicht zugänglich und verlangsamen damit (lokal) die Degradation. In der Folge kann es zur Fragmentbildung oder - durch die entsprechend verlängerte Anwesenheit des Implantats im Gewebe - zur Erhöhung der Restenoserate kommen. Zwar ist es prinzipiell denkbar, das Degradationsverhalten von Implantatsgrundkörper und aktiver Beschichtung durch Variation des Materials von Trägermatrix und Grundkörper, der Schichtdicke der aktiven Beschichtung, des Designs des Grundkörpers und ggf. der Zusammensetzung der Trägermatrix (Gehalt an pharmazeutisch aktiver Substanz, Hilfsstoffen) für ein konkretes Implantat zu optimieren, jedoch ist dies sehr aufwändig und die Ergebnisse sind nicht ohne weiteres auf Neuentwicklungen übertragbar.

Ein weiteres Problem ist der Einfluss des Prozesses der Degradation des Implantatsgrundkörpers auf die Freisetzung der pharmazeutisch aktiven Substanz aus der Trägermatrix. Die Abbauprodukte des Grundkörpers können sowohl die Freisetzung der Substanz aus der Trägermatrix als auch den Abbau der Trägermatrix - und damit indirekt wiederum die Freisetzung der Substanz - beeinflussen. Mit anderen Worten, die drei Prozesse (i) Freisetzung der Substanz, (ii) Abbau der Trägermatrix und (iii) Abbau des Implantatsgrundkörpers interagieren und die lokale Koinzidenz der Prozesse erschwert eine Optimierung des Implantats.

Der vorliegenden Erfindung liegt daher insbesondere die Aufgabe zugrunde, einen Weg zur Lösung oder zumindest Minderung der geschilderten Probleme aufzufinden.

Erfindungsgemäß wird diese Aufgabe durch den Stent mit einem Grundkörper aus einem biodegradierbaren Material und einer auf dem Grundkörper aufgebrachten aktiven Beschichtung, die eine biodegradierbare Trägermatrix und zumindest eine in die Trägermatrix eingebettete pharmazeutisch aktive Substanz umfasst, nach Anspruch 1 gelöst. Der Stent zeichnet sich dadurch aus, dass
(i) eine Abbaugeschwindigkeit der aktiven Beschichtung geringer ist als eine Abbaugeschwindigkeit des Grundkörpers; und
(ii) die aktive Beschichtung auf einem hierzu vorgesehenen Beschichtungsbereich der Oberfläche des Grundkörpers derart aufgebracht ist, dass
   - der Beschichtungsbereich in einen unbeschichteten Teilbereich und einen mit der aktiven Beschichtung beschichten Teilbereich gegliedert ist, wobei der beschichtete Teilbereich 5 bis 80 % der Oberfläche des Beschichtungsbereichs bedeckt;
   - ein Abstand von einem beliebigen Punkt der Oberfläche im beschichteten Teilbereich zum nächstgelegenen unbeschichteten Teilbereich kleiner ist als 60 µm; und
   - ein Abstand von einem beliebigen ersten Randpunkt der Oberfläche im beschichteten Teilbereich zu dem einen zweiten Randpunkt im selben beschichteten Teilbereich, der von dem ersten Randpunkt am weitesten entfernt ist, höchstens 400 µm beträgt.

Der Erfindung liegt die Erkenntnis zugrunde, dass ein im vorgesteckten Rahmen flächenmäßig begrenztes Aufbringen der aktiven Beschichtung im dazu vorgesehenen Beschichtungsbereich sowie eine Anpassung des Beschichtungsmusters unter Beachtung des vorgegebenen Abstands vor allem zu einer Entflechtung der Degradationsprozesse von Trägermatrix und Grundkörper führt. Hierdurch ist es möglich, die Freisetzung der pharmazeutisch aktiven Substanz sowie Vorgänge bei der Degradation genauer abzustimmen und ggf. notwendige Modifikationen nur auf einen Teil des Systems zu beschränken. Aufgrund der Grundgerüstdegradation werden sich die beschichteten Teilbereiche von der Oberfläche des Grundkörpers lösen und, falls im Gewebskontakt befindlich, in das umgebende Gewebe einwachsen. Dort fungieren sie als lokale Wirkstoffdepots, die weder lokal noch bezüglich der Freisetzungs- und Degradationsprozesse im Kontakt mit dem Grundgerüst des Implantats stehen.

Eine bevorzugte Ausgestaltungsform der Erfindung sieht vor, dass eine Freisetzungsgeschwindigkeit der pharmazeutisch aktiven Substanz größer als die Abbaugeschwindigkeit der Trägermatrix, jedoch geringer als die Abbaugeschwindigkeit des Grundkörpers ist. Hierdurch kann eine genauere Einstellung der Dosierung der pharmazeutisch aktiven Substanz in den vom Therapieplan festgelegten Bereichsgrenzen erfolgen, denn störende Interaktionen zu den Abbauprozessen des Implantatsgrundkörpers und der Trägermatrix werden vermieden oder zumindest gemindert. Vorzugsweise beträgt dabei die Freisetzungsgeschwindigkeit mindestens das zweifache der Abbaugeschwindigkeit der Trägermatrix, so dass die Menge an Substanz, die durch Diffusionsprozesse aus der Trägermatrix und nicht infolge der Zersetzung der Trägermatrix freigesetzt wird, erhöht ist. Ein Vorteil liegt darin, dass die durch Diffusion freigesetzte Substanz zumeist in einer für die Resorption im Körper adäquateren Modifikation vorliegt. Weiterhin ist aufgrund einer geminderten Interaktion zwischen den genannten Prozessen eine Modifikation des System, beispielsweise zur Anpassung an einen individuellen Therapieplan, vereinfacht.

Vorzugsweise beträgt die Abbaugeschwindigkeit des Grundkörpers das 1,1 bis 50fache der Abbaugeschwindigkeit der aktiven Beschichtung. Bei einer Abbaugeschwindigkeit unterhalb der genannten Bereichgrenzen erhöht sich die Gefahr unerwünschter Interaktionen zwischen den beiden Abbauprozessen. Bei einer Abbaugeschwindigkeit oberhalb der genannten Bereichgrenzen verlängert sich die Verweildauer der aktiven Beschichtungsteile im Körper erheblich, so dass Abstoßungsreaktionen wahrscheinlicher werden.

Vorzugsweise bedeckt der beschichtete Teilbereich 5 bis 20 % der Oberfläche des Beschichtungsbereichs. Oberhalb der genannten Grenze ist eine Angriffsfläche für das Körpermedium soweit verringert, dass es zu einer merklichen Verzögerung des Grundkörperabbaus im Beschichtungsbereich kommt und sich damit eine Interaktion der genannten Prozesse verstärken kann.

Der Abstand von einem beliebigen Punkt der Oberfläche im beschichteten Teilbereich zum nächstgelegenen unbeschichteten Teilbereich ist vorzugsweise kleiner ist als 30 µm.

Oberhalb der genannten Grenze besteht die Gefahr, dass der beschichtete Teilbereich lokal den Abbau des Grundgerüst verzögert - nämlich genau dort, wo der Abstand zum Rand des beschichteten Teilbereichs zu groß ist. In der Folge kann es zur Artefaktbildung kommen und ein Einwachsen der aktiven Beschichtung und agieren derselben als Wirkstoffdepot ist behindert.

Der Abstand von einem beliebigen ersten Randpunkt der Oberfläche im beschichteten Teilbereich zu dem einen zweiten Randpunkt, der von dem ersten Randpunkt am weitesten entfernt ist, beträgt vorzugsweise höchstens 200 µm, insbesondere höchstens 100 µm. Oberhalb der genannten Grenze besteht die Gefahr, dass der beschichtete Teilbereich lokal den Abbau des Grundgerüst verzögert. In der Folge kann es zur Artefaktbildung kommen und ein Einwachsen der aktiven Beschichtung und agieren derselben als Wirkstoffdepot ist behindert.

Die aktive Beschichtung besteht ferner vorzugsweise aus einer Vielzahl von Beschichtungsinseln. Diese weisen vorzugsweise einen mittleren Durchmesser von 10 bis 100 µm auf. Durch die Konturgebung als auch deren Durchmesserbegrenzung lässt sich der Herstellungsprozess besonders einfach gestalten und Modifikationen, z. B. zur Anpassung der Dosierung der aktiven Substanz, werden leichter ermöglicht.

Vorzugsweise ist der unbeschichtete Teilbereich in eine Vielzahl von Teilflächen gegliedert. Weiterhin nehmen Teilflächen mit einer Größe von bis zu 1000 µm² mindestens 70 % der gesamten Fläche des unbeschichteten Teilbereichs ein. Hierdurch wird sichergestellt, dass eine Angriffsfläche für das Körpermedium in dem unbeschichteten Teilbereich hinreichend groß ist, so dass eine Benetzung mit dem Körpermedium erleichtert ist. Ansonsten kann es zu einer merklichen Verzögerung des Grundkörperabbaus im Beschichtungsbereich kommen.

Vorzugsweise ist das Grundgerüst des Stents aus einer Magnesium-, Eisen- oder Wolframlegierung geformt. Besonders bevorzugt sind Magnesiumlegierungen vom Typ WE, insbesondere WE43. Letztere Legierung zeichnet sich durch die Anwesenheit von Seltenerden und Yttrium aus. Die genannten Werkstoffe lassen sich einfach verarbeiten, haben geringe Materialkosten und eignen sich aufgrund der relativ raschen Degradation und des gegenüber Polymeren günstigeren elastischen Verhaltens (geringerer Recoil des Stents) besonders für Gefäßstützen. Weiterhin wurde zumindest für einen Teil der Legierungen ein positiver physiologischer Effekt der Abbauprodukte auf den Heilungsprozess festgestellt. Ferner hat es sich gezeigt, dass aus WE43 hergestellte Magnesiumstents keine störenden Magnetresonanz-Artefakte erzeugen, wie sie beispielsweise vom medizinischen Edelstahl (316A) bekannt sind, und daher kann ein Therapieerfolg mit auf Magnetresonanz basierenden Detektionsgeräten verfolgt werden. Die biodegradierbaren Metalllegierungen aus den Elementen Magnesium, Eisen oder Wolfram enthalten vorzugsweise die genannten Elemente in einem Anteil von mindestens 50 Gew%, insbesondere mindestens 70 Gew%, besonders bevorzugt mindestens 90 Gew% an der Legierung.

Die Erfindung wird nachfolgend anhand eines Ausführungsbeispiels und den dazugehörigen Zeichnungen erläutert.
- Fig. 1: eine schematische Draufsicht auf einen Ausschnitt eines biodegradierbaren Implantats mit einer erfindungsgemäßen Beschichtung;
- Fig. 2: einen Schnitt durch den Grundkörper des Stents im Bereich der aktiven Beschichtung.

Der Begriff "biodegradierbar" bezieht sich vorliegend auf ein Material, das in-vivo abgebaut wird, also seine mechanische Integrität verliert. Nicht notwendigerweise müssen die Abbauprodukte vollständig vom Körper resorbiert oder ausgeschieden werden. Es können beispielsweise auch kleine Partikel am Ort der Applikation verbleiben. Biodegradation im erfindungsgemäßen Sinne betrifft insbesondere hydrolytische, enzymatische und andere stoffwechselbedingte Abbauprozesse im lebenden Organismus, die zu einer allmählichen Auflösung zumindest großer Teile der verwendeten Materialien führen. Synonym wird häufig der Begriff Biokorrosion verwendet. Der Begriff Bioresorption umfasst zusätzlich die anschließende Resorption der Abbauprodukte.

Eine "aktive Beschichtung" im Sinne der Erfindung umfasst zumindest eine biodegradierbare Trägermatrix und wenigstens eine in dieselbige eingebettete pharmazeutisch aktive Substanz. Optional kann die aktive Beschichtung noch weitere Hilfsstoffe enthalten, um beispielsweise Haftvermögen und Verarbeitbarkeit sowie die Freisetzung der Substanz zu verbessern. Als Materialien für die Trägermatrix kommen insbesondere Polymere natürlichen Ursprungs in Frage, z.B. Hyaluronsäure, Poly-L-Lactid, Poly-D-Lactid, Collagen und dergleichen.

Die erfindungsgemäß eingesetzte Trägermatrix basiert vorzugsweise auf einem biodegradierbaren Polymer. Biodegradierbare Polymere sind schon länger bekannt, und werden auch für orale Applikationen und Injektionen verwendet. Inzwischen werden viele unterschiedliche Polymerklassen für medizinische Zwecke eingesetzt, die jeweils für den entsprechenden Einsatz maßgeschneiderte Eigenschaften besitzen. Das verwendete Polymersystem muss auf seine physiologische Wirkung hin untersucht werden; seine Abbauprodukte dürfen nicht toxisch sein bzw. durch Reaktion mit körpereigenen Stoffen toxische Substanzen bilden. Zu beachten ist weiterhin, dass ein Potential der Polymersysteme zur Initiation von Infektionen aufgrund von Fremdkörperreaktionen des Immunsystems möglichst gering ist. Schließlich muss eine Wechselwirkung zwischen dem Wirkstoff und der Polymermatrix berücksichtigt werden: so dürfen die Polymere durch Wechselwirkung mit dem Wirkstoff weder ihre biodegradierbaren Eigenschaften verlieren noch darf durch Reaktion des Wirkstoffs mit der Polymermatrix eine Deaktivierung des Wirkstoffs stattfinden. Bei der Auswahl eines spezifischen Systems aus Polymermatrix und Wirkstoff wird der Fachmann daher die genannten Parameter berücksichtigen.

Eine "pharmazeutisch aktive Substanz" im Sinne der Erfindung ist ein pflanzlicher, tierischer oder synthetisierter Wirkstoff, der in geeigneter Dosierung als Therapeutika zur Beeinflussung von Zuständen oder Funktionen des Körpers, als Ersatz für natürlich vom menschlichen oder tierischen Körper erzeugte Wirkstoffe sowie zur Beseitigung oder zum Unschädlichmachen von Krankheitserregern oder Körperfremdstoffen Einsatz findet. Die Freisetzung der Substanz in der Implantatsumgebung hat einen positiven Effekt auf den Heilungsverlauf bzw. wirkt pathologischen Veränderungen des Gewebes infolge des chirurgischen Eingriffes entgegen.

Unter "Freisetzung der pharmazeutisch aktiven Substanz" wird das Entfernen der Substanz aus der Trägermatrix verstanden. Ein Teilprozess ist dabei das Herauslösen absorbierter Substanz aus der festen oder gelartigen Trägermatrix mit Hilfe von körperpräsenten Medien, wie Blut.

Eine Freisetzungsgeschwindigkeit wird wie folgt bestimmt: es wird eine Halbwertzeit erfasst, in der 50 Gew.% der Substanz freigesetzt sind und anhand dieser Halbwertzeit wird dann bei unterstellter linearer Freisetzungskinetik eine (mittlere) Freisetzungsgeschwindigkeit bestimmt.

Eine Abbaugeschwindigkeit der Trägermatrix und des Grundkörpers wird derart erfasst, dass zunächst eine Halbwertzeit ermittelt wird, bei der 50 Gew.% des den Grundkörper bilden Materials bzw. der Trägermatrix abgebaut sind und dann anhand dieser Halbwertzeit bei unterstelltem linearen Verlauf des Abbaus eine (mittlere) Geschwindigkeit des Abbauprozesses berechnet wird.

Das Grundgerüst des Stent umfasst alle zur Gewährleistung der mechanischen Integrität und Grundfunktionalitäten des Implantats notwendigen Bauelemente. Zusätzlich kann der Stent z. B. Markerelemente aufweisen, die mit dem Grundkörper in geeigneter Weise verbunden sind. Das Grundgerüst stellt eine Oberfläche bereit, die zur Auftragung der aktiven Beschichtung dient. Ein Bereich der Beschichtung kann individuell festgelegt werden; vorzugsweise wird dabei nur ein nach außen gerichteter Teil des Grundgerüst beschichtet.

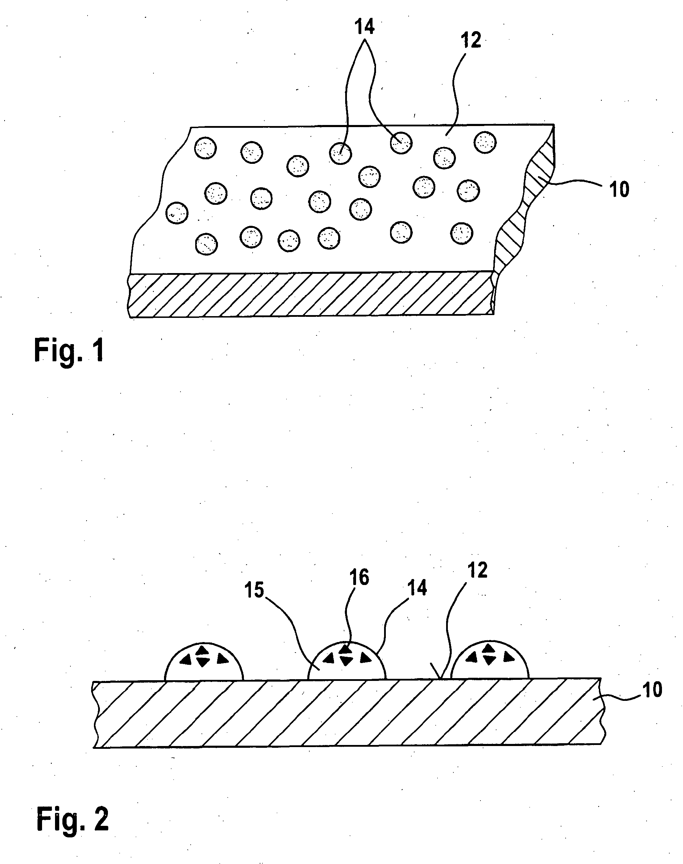

Fig. 1 zeigt einen Abschnitt aus einem Grundkörper 10 eines Stents, der aus einem biodegradierbaren Material geformt ist. Der metallische Werkstoff bildet ein filigranes Gerüst aus miteinander verbundenen Streben, deren Design für die vorliegende Erfindung nur von untergeordneter Bedeutung ist. Auf eine äußere Oberfläche 12 des Grundkörpers 10 wird eine aktive Beschichtung aufgebracht. Wie ersichtlich, ist der Beschichtungsbereich in einen unbeschichteten Teilbereich und einen mit der aktiven Beschichtung beschichten Teilbereich gegliedert.

Die aktive Beschichtung ist vorliegend als eine Vielzahl von Beschichtungsinseln 14 realisiert, die aus einer biodegradierbaren Trägermatrix 15 und zumindest einer in die Trägermatrix 15 eingebetteten pharmazeutisch aktiven Substanz 16 (hier dargestellt als Dreieck) bestehen. Die Beschichtungsinseln 14 sind derart auf der Oberfläche 12 des Grundkörpers 10 aufgebracht, dass der beschichtete Teilbereich, also die Beschichtungsinseln 14, etwa 10 - 15 % der Oberfläche 12 des Beschichtungsbereichs bedecken.

Der Grundkörper 10 besteht vorliegend aus der Magnesiumlegierung WE43 und die Trägermatrix ist hochmolekulares Poly-L-Lactid (Molmasse > 500 kD). Eine Abbaugeschwindigkeit des Materials des Grundkörpers 10 beträgt etwa das 10 - 15fache im Vergleich zur Abbaugeschwindigkeit des polymeren Materials der Trägermatrix 15.

Die einzelnen Beschichtungsinseln weisen einen mittleren Durchmesser von etwa 50 bis 70 µm auf. Ein Abstand eines beliebigen Punktes der Oberfläche im beschichteten Teilbereich zum nächstgelegenen unbeschichteten Teilbereich ist somit kleiner ist als 35 µm. Sind die Beschichtungsinseln gleichförmig rund, so liegt der Abstand von einem beliebigen ersten Randpunkt der Oberfläche im beschichteten Teilbereich zu einen zweiten Randpunkt, der von dem ersten Randpunkt am weitesten entfernt ist, bei etwa 50 bis 70 µm.

Zur Aufbringung der Beschichtungsinseln 14 kann wie folgt vorgegangen werden:

Der Stent wird auf einen Ballon oder Katheter vormontiert. In einem Reservoir wird eine Lösung/Feinstdispersion des biodegradierbaren Polymers und der wenigstens einen aktiven Substanz bereitgestellt. Über ein ansteuerbares Mikroinjektionssystem werden anschließend Tropfen definierter Größe in ausgewählten Bereichen des Grundkörpers aufgetragen. Durch Verdampfen wird das Lösungsmittel entzogen und es bilden sich die Beschichtungsinseln definierten Durchmessers aus.

## Patentansprüche

1. Stent mit einem Grundkörper aus einem biodegradierbaren Material und einer auf dem Grundkörper aufgebrachten aktiven Beschichtung, die eine biodegradierbare Trägermatrix und zumindest eine in die Trägermatrix eingebettete pharmazeutisch aktive Substanz umfasst, wobei die Abbaugeschwindigkeit der aktiven Beschichtung geringer ist als eine Abbaugeschwindigkeit des Grundkörpers, **dadurch gekennzeichnet, dass**
die aktive Beschichtung auf einem hierzu vorgesehenen Beschichtungsbereich der Oberfläche des Grundkörpers derart aufgebracht ist, dass
- der Beschichtungsbereich in einen unbeschichteten Teilbereich und einen mit der aktiven Beschichtung beschichten Teilbereich gegliedert ist, wobei der beschichtete Teilbereich 5 bis 80% der Oberfläche des Beschichtungsbereichs bedeckt;
- ein Abstand von einem beliebigen Punkt der Oberfläche im beschichteten Teilbereich zum nächstgelegenen unbeschichteten Teilbereich kleiner ist als 35 µm; und
- ein Abstand von einem beliebigen ersten Randpunkt der Oberfläche im beschichteten Teilbereich zu dem einem zweiten Randpunkt im selben beschichteten Teilbereich, der von dem ersten Randpunkt am weitesten entfernt ist, höchstens 400 µm beträgt.

2. Stent nach Anspruch 1, bei dem die Abbaugeschwindigkeit des Grundkörpers das 1,1 bis 50fache der Abbaugeschwindigkeit der aktiven Beschichtung beträgt.

3. Stent nach Anspruch 1, bei dem der beschichtete Teilbereich 5 bis 20% der Oberfläche des Beschichtungsbereichs bedeckt.

4. Stent nach Anspruch 1, bei dem der Abstand von einem beliebigen Punkt der Oberfläche im beschichteten Teilbereich zum nächstgelegenen unbeschichteten Teilbereich kleiner ist als 30 µm.

5. Stent nach Anspruch 1, bei dem eine Freisetzungsgeschwindigkeit der pharmazeutisch aktiven Substanz größer als die Abbaugeschwindigkeit der Trägermatrix, jedoch geringer als die Abbaugeschwindigkeit des Grundkörpers ist.

6. Stent nach Anspruch 1, bei dem die aktive Beschichtung aus einer Vielzahl von Beschichtungsinseln besteht.

7. Stent nach Anspruch 6, bei dem die Beschichtungsinseln einen mittleren Durchmesser von 10 bis 100 µm aufweisen.

8. Stent nach Anspruch 1, bei dem der unbeschichtete Teilbereich in eine Vielzahl von Teilflächen gegliedert ist, und Teilflächen mit einer Größe von bis zu 1000 µm² mindestens 70 % der gesamten Fläche des unbeschichteten Teilbereichs einnehmen.

## Claims

1. A stent having a main body formed from a biodegradable material, and an active coating applied to the main body, the coating comprising a biodegradable carrier matrix and at least one pharmaceutically active substance embedded in the carrier matrix, wherein the degradation rate of the active coating is lower than a degradation rate of the main body,
**characterised in that**
the active coating is applied to a coating region of the surface of the main body provided for this purpose such that
- the coating region is divided into an uncoated partial region and a partial region coated with the active coating, wherein the coated partial region covers 5 to 80 % of the surface of the coating region;
- a distance from an arbitrary point of the surface in the coated partial region to the closest uncoated partial region is less than 35 µm; and
- a distance from an arbitrary first edge point of the surface in the coated partial region to a second edge point in the same coated partial region, which is furthest away from the first edge point, is at most 400 µm.

2. The stent according to Claim 1, wherein the degradation rate of the main body is 1.1 to 50 times the degradation rate of the active coating.

3. The stent according to Claim 1, wherein the coated partial region covers 5 to 20 % of the surface of the coating region.

4. The stent according to Claim 1, wherein the distance from an arbitrary point of the surface in the coated partial region to the closest uncoated partial region is less than 30 µm.

5. The stent according to Claim 1, wherein release rate of the pharmaceutically active substance is greater than the degradation rate of the carrier matrix, but lower than the degradation rate of the main body.

6. The stent according to Claim 1, wherein the active coating consists of a multiplicity of coating islands.

7. The stent according to Claim 6, wherein the coating islands have a mean diameter from 10 to 100 µm.

8. The stent according to Claim 1, wherein the uncoated partial region is divided into a multiplicity of partial surfaces having a size of up to 1000 µm² of at least 70 % of the total surface of the uncoated partial region.

## Revendications

1. Stent avec un corps de base constitué d'un matériau biodégradable et d'un revêtement actif appliqué sur le corps de base, lequel comprend une matrice de support biodégradable et au moins une substance pharmaceutique active enrobée dans la matrice de support, dans lequel la vitesse de dégradation du revêtement actif est inférieure à une vitesse de dégradation du corps de base,
**caractérisé en ce que**
le revêtement actif est appliqué de telle manière sur une zone de revêtement prévue à cet effet sur la surface du corps de base, que
- la zone de revêtement est divisée en une zone partielle sans revêtement et une zone partielle revêtue du revêtement actif, la zone partielle revêtue couvrant 5 à 80% de la surface de la zone de revêtement ;
- un espacement entre un point au choix de la surface dans la zone partielle revêtue et la zone partielle non revêtue la plus proche est inférieur à 35 µm ; et
- un espacement entre un premier point de bord au choix de la surface dans la zone partielle revêtue et le deuxième point de bord le plus éloigné du premier point de bord dans la même zone partielle revêtue mesure tout au plus 400 µm.

2. Stent selon la revendication 1, dans lequel la vitesse de dégradation du corps de base mesure de 1,1 à 50 fois la vitesse de dégradation du revêtement actif.

3. Stent selon la revendication 1, dans lequel la zone partielle revêtue couvre entre 5 et 20% de la surface de la zone de revêtement.

4. Stent selon la revendication 1, dans lequel l'espacement entre un point au choix de la surface dans la zone partielle revêtue et la zone partielle non revêtue la plus proche est inférieur à 30 µm.

5. Stent selon la revendication 1, dans lequel la vitesse de libération de la substance pharmaceutique active est supérieure à la vitesse de dégradation de la matrice de support, mais inférieure à la vitesse de dégradation du corps de base.

6. Stent selon la revendication 1, dans lequel le revêtement actif est constitué d'une pluralité d'îlots de revêtement.

7. Stent selon la revendication 6, dans lequel les îlots de revêtement présentent un diamètre moyen de 10 à 100 µm.

8. Stent selon la revendication 1, dans lequel la zone partielle non revêtue est divisée en une pluralité de surfaces partielles, et les surfaces partielles avec une taille allant jusqu'à 1000 µm² représentent au moins 70% de la surface totale de la zone partielle non revêtue.
